# EUROPEAN PATENT SPECIFICATION

(11) **EP 3 054 795 B1**
(45) Date of publication and mention of the grant of the patent: **27.01.2021**
(21) Application number: 14781210.1
(22) Date of filing: 07.10.2014
(51) Int. Cl.: A24D 3/04

(54) **AEROSOL TRANSFERRING ADAPTER FOR AN AEROSOL GENERATING DEVICE AND METHOD FOR TRANSFERRING AEROSOL WITHIN AN AEROSOL GENERATING DEVICE**
AEROSOLÜBERFÜHRUNGSADAPTER FÜR EINE AEROSOLERZEUGUNGSVORRICHTUNG UND VERFAHREN ZUM ÜBERRAGEN VON AEROSOL MITHILFE DER AEROSOLERZEUGUNGSVORRICHTUNG
ADAPTATEUR DE TRANSFERT D'AÉROSOL DESTINÉ À UN DISPOSITIF DE GÉNÉRATION D'AÉROSOL ET PROCÉDÉ DE TRANSFERT D'AÉROSOL À L'INTÉRIEUR D'UN DISPOSITIF DE GÉNÉRATION D'AÉROSOL

(30) Priority: 08.10.2013 EP 13187706
(43) Date of publication of application: 17.08.2016
(73) Proprietor: JT International S.A., 1211 Geneva 26 (CH)
(72) Inventor: ROGAN, Andrew Robert John, Forres Moray Central Scotland IV36 2HL (GB); SMITH, Arlene Deborah, Doagh Antrim Antrim BT39 ORB (GB)
(74) Representative: Hoffmann Eitle
(86) International application number: PCT/EP2014/071453
(87) International publication number: WO 2015/052192

(56) References cited:
- EP-A1- 0 317 154
- WO-A1-2013/083636
- WO-A1-2013/127810
- US-A- 2 124 130
- US-A1- 2013 160 764

## Description

### Field of the invention

The present invention pertains to an aerosol transferring adapter for an aerosol generating device, an aerosol generating device comprising such an aerosol transferring adapter and a method for transferring aerosol within an aerosol generating device, in particular for use with refillable electronic cigarettes or vaporizers.

### Background of the invention

Conventional electrically operated cigarettes, so-called "e-cigarettes", usually include a heater powered by an electrical power source and a liquid reservoir containing flavoured liquid that can be volatilized using the heater and transferred to a user of the e-cigarette in an airflow through a mouthpiece of the e-cigarette.

Such electrically operated cigarettes are for example known from the patent documents WO 2013/127810 A1, US 2013/0160764 A1, and WO 2013/083636 A1.

With existing e-cigarettes it is possible for a user to choose one specific flavoured liquid to produce a corresponding flavoured aerosol at a time. It is unfortunately not possible for the user to choose, with one same e-cigarette, between several different flavoured liquids and produce several different flavoured aerosols among which he can choose prior or during use according to his taste and mood. To be able to change the aerosol flavour he enjoys the user needs to change the aerosol generating liquid in his device, which is quite cumbersome and unhygienic due to potential risks of leakage of the liquid in and out of the device and the liquid reservoir/cartridge holding it.

### Summary of the invention

It is therefore one object of the present invention to provide disposable or reusable mouthpieces for aerosol generating devices which enable a user of the aerosol generating devices to flexibly adjust the taste profile and properties of the inhaled aerosol.

This problem is solved by a mouthpiece for an aerosol generating device having the technical features of claim 1, an aerosol transferring adapter having the technical features of claim 7, an aerosol generating device having the technical features of claim 12, and a method for transferring aerosol within an aerosol generating device having the technical features of claim 15.

According to a first aspect of the present invention, a mouthpiece comprises at least two mouthpiece compartments being fluidly separated from each other and each leading from at least one mouthpiece inlet to at least one mouthpiece outlet, and a mouthpiece fitting arranged around the mouthpiece inlet and configured to be alignably connected to an aerosol generating device.

According to a second aspect of the present invention, an aerosol transferring adapter for an aerosol generating device comprises a liquid storage component (for example, absorbent plastic mesh, or storage tank) and a mouthpiece according to the first aspect of the invention.

The liquid storage component comprises a liquid reservoir for holding a liquid used to generate an aerosol, at least one airflow channel leading through the liquid reservoir, and a connector fitting arranged around the airflow channel. The mouthpiece fitting is configured to be alignably connected to the connector fitting of the liquid storage component.

According to a third aspect of the present invention, an aerosol generating device, comprising an aerosol transferring adapter according to the second aspect, and a heating element coupled to the liquid reservoir at a liquid interface and configured to heat liquid in the liquid reservoir, thereby generating aerosol in the airflow channel.

According to a fourth aspect of the present invention, a method for transferring aerosol within an aerosol generating device comprises the following steps: generating aerosol by volatilizing compounds of a liquid composition in a liquid reservoir of the aerosol generating device; guiding the generated aerosol to a mouthpiece of the aerosol generating device, the mouthpiece comprising at least two mouthpiece compartments being fluidly separated from each other and each leading from a mouthpiece inlet to a mouthpiece outlet; aligning the position of the mouthpiece with respect to the liquid reservoir; and guiding the generated aerosol through at least one of the mouthpiece compartments depending on the aligned position of the mouthpiece with respect to the liquid reservoir (or area that electronic cigarette is attached to.)

### Embodiments of the invention

One main idea of the present invention is to provide a disposable mouthpiece component for an aerosol generating device that can be connected to the aerosol generating device and at the same time be aligned in different positions with respect to the main body of the aerosol generating device in order to align different flow-through channels of the mouthpiece component with an outlet of the aerosol generating device.

One advantage of such component is the possibility to modify properties of the airflow with the aerosol of the aerosol generating device through the mouthpiece component by virtue of varying properties of the respectively aligned flow-through channel without having to change the aerosol generating device itself. A user can select and adjust the aerosol taste, flow-through rate or other properties for example by twisting or mechanically adjusting the connecting position of the mouthpiece component with respect to the aerosol generating device. Particularly advantageous is the fact that varying aerosol modification tools such as filtration materials, tobacco or tobacco derivatives, flavour matrices or moisture pads may be incorporated in the mouthpiece component without the need to alter the aerosol generating device.

Especially for flavours, it is advantageous that the flavours in the mouthpiece component are more stable since they neither get into contact with other ingredients used to produce the aerosol, for example the source liquid, nor do they need to be directly heated. This prevents the formation of unwanted pyrolysis materials, prevents the deposition of non-volatile components of natural flavours on the heating element and helps prolonging the flavour generating properties of the mouthpiece component.

Inclusion of tobacco or tobacco derivatives as an aerosol modification component can help with improvement of the taste and sensory characteristics of the aerosol generating device, such that the characteristics become more similar to those of traditional combustible tobacco products.

Filtration materials may also be used for the mouthpiece component in order to remove the amount of visible smoke emitted from the aerosol generating device or to remove undesirable components, particles or residues.

The dependent claims provide additional technical features of advantageous embodiments and further improvements of the invention.

According to an embodiment of the aerosol transferring adapter, the aerosol transferring adapter further comprises at least one sealing ring fitted between the connector fitting and the mouthpiece fitting fluidly sealing the interface between the liquid storage component and the mouthpiece against the environment. Thereby, unwanted in flowing air or particles from outside may be blocked out, so that the aerosol altering properties of the mouthpiece compartments may be as predictable and stable as possible.

According to a further embodiment of the aerosol transferring adapter, the connector fitting and the mouthpiece fitting realize complementary snap-fit parts or interlocking clearances. This has the advantage, that the liquid storage component and the mouthpiece may be connected in a manner that is very difficult to reverse without breaking the adapter. Therefore, contact with substances in the liquid reservoir as well as undesired user initiated manipulations to the adapter may be avoided.

According to a further embodiment of the aerosol transferring adapter, at least one of the at least two mouthpiece compartments comprises an aerosol modifying medium arranged therein. According to one possibility, the aerosol modifying medium may comprise a flavourant, tobacco, tobacco derivative(s) or a combination thereof. Alternatively or additionally, the aerosol modifying medium may comprise a filtration material. Further alternatively or additionally, the aerosol modifying medium may comprise a moisturizing/drying pad.

Further alternatively or additionally, the aerosol modifying medium may comprise tobacco or a tobacco derivative. Characteristics of the tobacco or tobacco derivative, such as moisture, pH value or particle size distribution, can be altered to enable control of the aerosol taste and sensory characteristics. For example, a smaller average particle size may permit better release of tobacco flavour components by increasing the surface area. However, smaller particles may also cause an increase in pressure drop through the tobacco medium.

The mouthpiece may be filled with varying aerosol modifying mediums for different purposes, thereby enhancing the flexibility in using the aerosol generating device without the need to alter its general functionality or having to use different aerosol generating devices.

According to a further embodiment of the aerosol transferring adapter, at least two of the mouthpiece compartments comprise different aerosol modifying mediums arranged therein. This has the advantage that a user may easily and flexibly change the properties of the generated aerosol during use of the aerosol generating device.

According to a further embodiment of the aerosol transferring adapter, the mouthpiece is rotatable against the liquid storage component, the rotation causing the airflow channel to connect to a different compartment of the at least two of the mouthpiece compartments depending on the rotation position of the mouthpiece. Especially for rotation, in the case of symmetrically constructed aerosol generating devices and corresponding aerosol transferring adapters, this alignment procedure allows for easy, comfortable and reliable handling without altering the outer shape of the device overall.

According to a further embodiment of the aerosol transferring adapter, the aerosol transferring adapter comprises one or more alignment marks in the outer housing of the mouthpiece and/or the liquid storage component configured to indicate the rotation position of the mouthpiece to a user of the aerosol generating device. Such alignment marks may be signs, colour coded marks or similar visual indicators.

Alternatively or additionally, locking, snapping or latching marks using recesses, clearances and/or tappets may be provided giving the user auditory or tactile feedback upon alignment of the mouthpiece.

According to an embodiment of the aerosol generating device, the aerosol generating device may further comprise an electrical power source coupled to the heating element and configured to provide the heating element with electrical energy.

According to a further embodiment of the aerosol generating device, the aerosol generating device may further comprise a housing enclosing at least the heating element and the electrical power source.

According to an embodiment of the method of the third aspect of the invention, the step(s) of guiding the generated aerosol comprises an inhaling action of a user of the aerosol generating device through the mouthpiece outlet.

The invention will be explained in greater detail with reference to exemplary embodiments depicted in the drawings as appended.

### Brief description of the drawings

The accompanying drawings are included to provide a further understanding of the present invention and are incorporated in and constitute a part of this specification.

The drawings illustrate the embodiments of the present invention and together with the description serve to explain the principles of the invention. Other embodiments of the present invention and many of the intended advantages of the present invention will be readily appreciated as they become better understood by reference to the following detailed description. The elements of the drawings are not necessarily to scale relative to each other. Like reference numerals designate corresponding similar parts.
Fig. 1 schematically illustrates a concept of an aerosol generating device according to an embodiment of the invention.
Fig. 2 schematically illustrates a functional depiction of an aerosol generating device according to a further embodiment of the invention.
Fig. 3 schematically illustrates a perspective enlargement view of an aerosol transferring adapter according to yet another embodiment of the invention.
Fig. 4 schematically illustrates a cross-sectional view of an aerosol transferring adapter according to yet another embodiment of the invention.
Fig. 5 schematically illustrates a perspective view of an aerosol transferring adapter according to yet another embodiment of the invention.
Fig. 6 schematically illustrates a method for transferring aerosol within an aerosol generating device according to yet another embodiment of the invention.

### Detailed description of the embodiments

Although specific embodiments have been illustrated and described herein, it will be appreciated by those of ordinary skill in the art that a variety of alternate and/or equivalent implementations may be substituted for the specific embodiments shown and described Generally, this application is intended to cover any adaptations or variations of the specific embodiments discussed herein. In the present invention, reference is made to aerosol generators. Such aerosol generators or aerosol generating device are generally intended to comprise any apparatus capable of converting electric energy and/or combustion energy into heat and subsequently heating and thereby volatilizing particles in a vaporisable material, for example, a liquid composition contained within a part of the aerosol generating device. Aerosol generating devices within the meaning of the present invention may transport the volatilized particles in an airflow through the aerosol generating device to a user of the device, the user of the device being able to activate or deactivate the generation of aerosol and to control the duration, velocity and volume of the airflow by means of puffing or inhaling action.

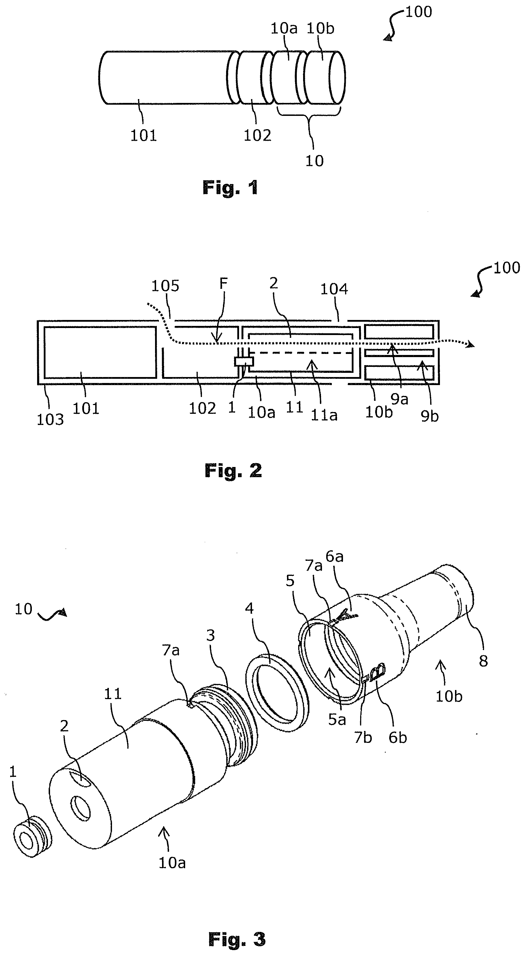

Fig. 1 schematically illustrates a concept of an aerosol generating device 100. The aerosol generating device 100 may generally comprise a power source 101, such as an electrical power source, for example a battery or an accumulator. The power source 101 is generally operatively coupled to a heating element 102, the heating element being supplied with energy for its operation by the power source 101. The power source 101 and the heating element 102 may be part of a basis of the aerosol generating device 100. The aerosol generating device 100 may further comprise electronic circuitry controlling the operation of the aerosol generating device 100 and/or various buttons or light emitting elements on its outer surface, all of which are not shown in Fig. 1 for purposes of improved clarity of the drawings.

The aerosol generating device 100 may comprise an aerosol transferring adapter 10 which may consist of two separable components 10a and 10b. The component 10a may be a liquid storage component which may be coupled to the heating element 102 in operative connection, so that the heating element 102 may supply heating energy to a liquid contained with the liquid storage component 10a, thereby causing certain compounds contained in the liquid to volatilize into an airflow streaming through the liquid storage component 10a. The liquid storage component 10a may be operatively coupled to a mouthpiece 10b in order to guide the generated aerosol in the liquid storage component 10a through the mouthpiece 10b towards a user of the aerosol generating device 100.

The overall arrangement of the power source 101, the heating element 102 and the aerosol transferring adapter 10 may deviate from the conceptual illustration in Fig. 1 and may be adapted to the design and purpose of the aerosol generating device 100. It may for example be possible to implement electrical connections between the aerosol generating device 100 and the aerosol transferring adapter 10 enabling control circuitry within the aerosol generating device 100 to gather information regarding the operational state of the aerosol transferring adapter 10.

Fig. 2 shows a schematic illustration of a functional depiction of an aerosol generating device 100. The aerosol generating device 100 may again comprise a power source 101 and a heating element 102 as explained in conjunction with Fig. 1.

The power source 101 and the heating element 102 may for example be enclosed in a housing 103 having an inlet 105 in the general vicinity of the heating element 102 in order to have air stream F flow through or along the heating element 102 towards the liquid storage component 10a. The liquid storage component 10a may comprise a liquid reservoir 11 in which a liquid composition 11a with compounds to be volatilized may be contained. The liquid reservoir 11 may be in connection with the heating element 102 by means of a liquid interface 1 which may convey heating energy to the liquid 11a, thereby evaporating or volatilizing certain compounds of the liquid 11a. The air stream or airflow F may enter the liquid reservoir 11, flow through an airflow channel 2 leading through the liquid reservoir 11 over or near the liquid 11a and absorb the volatilized or evaporated particles, thus creating an aerosol in the liquid reservoir 11.

The generated aerosol may then be transported in the airflow F to the mouthpiece 10b. The mouthpiece 10b may comprise at least two mouthpiece compartments 9a, 9b being fluidly separated from each other and each leading from a mouthpiece inlet to a mouthpiece outlet, the mouthpiece inlet being in fluid communication with the airflow channel 2 and the mouthpiece outlet being on the far end of the liquid storage component 10a. At the mouthpiece outlet, the aerosol guided through the mouthpiece 10b may be inhaled by a user of the aerosol generating device 100. The number of mouthpiece compartments 9a, 9b is not limited to two, but may be any suitable number as well.

The different mouthpiece compartments 9a, 9b may be used to modify or alter the properties of the aerosol being guided through the mouthpiece 10b. For example, one or more of the mouthpiece compartments 9a, 9b may be equipped with an aerosol modifying medium arranged therein. Such aerosol modifying mediums may for example comprise flavourants, for example flavourants adsorbed onto solid matrices, encapsulated flavourants, surface coated flavourant particles, tobacco, tobacco derivatives or similar materials. It may of course also be possible to include combinations of the aforementioned materials in the aerosol modifying mediums.

It may also be possible for the aerosol modifying medium to comprise a filtration material. The filtration material may for example be used to reduce the amount of visible aerosol or to remove unwanted particles or components from the airflow to the user. The aerosol modifying medium may also be used to change aerosol characteristics such as moisture, pH value, particle size or the like, for example in order to mimic the taste of traditional tobacco products as well as possible.

The aerosol modifying medium may for this purpose for example comprise a moisturizing/drying pad, a pH changing material or sieve means.

While the number, placement and amount of aerosol modifying mediums in the mouthpiece compartments 9a, 9b may vary, it may be preferred to equip at least two of the mouthpiece compartments 9a, 9b with different aerosol modifying mediums arranged therein. Depending on the compartment 9a, 9b through which the airflow F will stream, different modifiers might be used on the aerosol, thereby generating different inhaling experiences for the user. For example, it may be possible to have one of the mouthpiece compartments 9a, 9b equipped with a flavourant, while another of the mouthpiece compartments 9a, 9b may be left blank. In such case, by aligning the mouthpiece 10b with respect to the liquid storage component 10a, the airflow F may be selectively directed towards one of the two mouthpiece compartments 9a, 9b, thus allowing perfect control of the flavour and intensity of the modified aerosol. The mouthpiece compartments 9a, 9b can also be subdivided further, to facilitate the positioning and securing of the aerosol modification medium within the mouth piece compartments 9a, 9b.

The housing 103 may be interrupted by gripping recesses 104 so that the user is able to more easily create a torque between the liquid storage component 10a and the mouthpiece 10b in order to align the mouthpiece 10b in a desired position. Alternatively or additionally, the housing may have alignment notches or grooves fitting the liquid storage component 10b, thereby stopping undesired concomitant rotation of the liquid storage component 10b with the mouthpiece 10a inherently by design.

Fig. 3 to 5 show schematic illustrations of an aerosol transferring adapter 10 in greater detail. The aerosol transferring adapter 10 of Fig. 3 may for example be used for an aerosol generating device as shown and explained in Figs. 1 and 2.

The aerosol transferring adapter 10 comprises the liquid storage component 10a and a mouthpiece 10b. The liquid storage component comprises a liquid reservoir 11 for holding a liquid used to generate an aerosol. An airflow channel 2 leads through the liquid reservoir 11, and the liquid reservoir 11 may be connected to a heating element 102 of an aerosol generating device 100 by means of a liquid interface 1, for example a plastic or metal grommet.

The liquid storage component 10a also comprises a connector fitting 3 arranged around the airflow channel 2. Correspondingly, the mouthpiece 10b comprises a mouthpiece fitting 5 arranged around a mouthpiece inlet 5a and configured to be alignably connected to the connector fitting 3 of the liquid storage component 10a. The connector fitting 3 and the mouthpiece fitting 5 may be constructed in a complementary and corresponding manner, for example by a moulding procedure, to realize complementary snap-fit parts or interlocking clearances. For example, the mouthpiece fitting 5 may include protrusions that interlock with recesses in the connector fitting 3 when edging the tubular connector fitting 3 into the mouthpiece fitting 5.

Between the connector fitting 3 and the mouthpiece fitting 5 a sealing ring 4, for example a rubber O-ring or similar, may be fitted thereby fluidly sealing the interface between the liquid storage component 10a and the mouthpiece 10b against the environment.

The mouthpiece 10b may be manufactured as essentially cylindrical to be rotatable against the liquid storage component 10a, the rotation causing the airflow channel 2, which may be arranged off-centre in the liquid reservoir 11, to connect to a different compartment of the at least two of the mouthpiece compartments 9a, 9b, 9c, 9d depending on the rotation position of the mouthpiece 10b. The rotation position of the mouthpiece 10b may be indicated to a user of the aerosol generating device by one or more alignment marks 7a, 7b in the outer housing of the mouthpiece 10b and/or the liquid storage component 10a. The alignment marks 7a, 7b may for example be visual marks like colour codes or sign labels. Alternatively or additionally, the alignment marks 7a, 7b may be haptic marks like indentations, grooves, notches or bumps on the respective components to provide a tactile response to a user touching the alignment marks 7a, 7b. It may also be possible to arrange the alignment marks 7a, 7b to generate noise or sound, like clicks or snaps as audible confirmation of the rotation position of the mouthpiece 10b. Additionally, sign labels or operational mode indicators 6a, 6b may be arrange on the housing of the mouthpiece 10b and/or the liquid storage component 10a as well.

Additionally, the position of the rotatable mouthpiece 10b may be communicated to an electronic control circuit of the aerosol generating device 100, such that aerosol generating device 100 functionality can be modified to optimise performance with that particular flavour. For example, the colour of the light emitted during puffing could be matched with the flavour in use, such as the colour green for menthol flavour, or the heating profile of the heating element 102 could be altered to suit the chemical composition of the flavour.

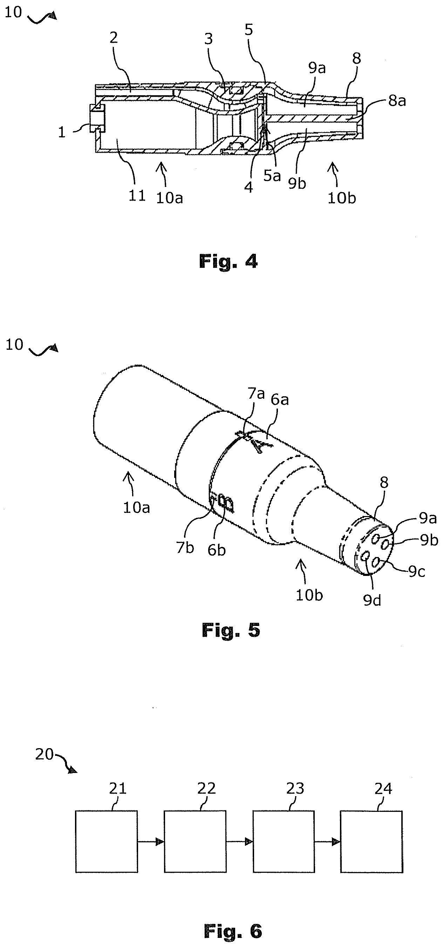

Fig. 4 shows a schematic illustration of a cross-sectional view of the aerosol transferring adapter 10 in Fig. 3. The mouthpiece compartments 9a, 9b of the mouthpiece 10b are arranged at a circumference around a centre partitioning element 8a, like a moulded wall. As shown in Fig. 4, the airflow channel 2 through the liquid reservoir 11 is designed to lie off-centre of the liquid reservoir 11. This configuration helps in ensuring proper and effective alignment of the mouthpiece compartments 9a, 9b with the airflow channel 2 upon rotation of the mouthpiece 10b. Depending on the tightness of the seal between the connector fitting 3 and the mouthpiece fitting 5 as well as on the distance between the partitioning element 8a and the wall of the liquid reservoir 11, the portion of the airflow F being guided from the airflow channel 2 through the respectively aligned mouthpiece compartment 9a, 9b will vary. For example, if the partitioning element 8a completely seals off the remaining momentarily non-aligned mouthpiece compartments from the airflow channel 2, all the aerosol generated in the liquid reservoir 11 will flow through momentarily the aligned mouthpiece compartment (compartment 9a in the position shown in Fig. 3, as an example).

When rotating the mouthpiece 10b by 180° with respect to the liquid reservoir 11, the respectively other mouthpiece compartment (compartment 9b in the position shown in Fig. 3, as an example) will align with the airflow channel 2. This means that a user is able to use different mouthpiece compartments 9a, 9b for transferring the aerosol from the liquid reservoir 11 to his mouth. Depending on the properties of the aerosol modifying mediums in the different mouthpiece compartments 9a, 9b the user is able to easily change flavour type, flavour intensity, particle size, moisture or other properties of the airflow F to be inhaled.

Fig. 5 shows another perspective view of the aerosol transferring adapter 10 in assembled state. The alignment marks 7a, 7b are in congruence with each other, with the mouthpiece 10b being rotated into operational state "A", as exemplarily indicated by the alignment indicators 6a (and/or 6b). The exemplary aerosol transferring adapter 10 of Fig. 5 is equipped with four mouthpiece compartments 9a, 9b, 9c, 9d having the outlet at the mouthpiece outlet 8.

Fig. 6 schematically illustrates a method 20 for transferring aerosol within an aerosol generating device, such as the aerosol generating device 100 of Fig. 1 and 2. The method may make use of an aerosol transferring adapter, for example an aerosol transferring adapter 10 as shown and described in conjunction with Figs. 3 to 5. The method 20 may comprise as first step generating aerosol 21 by volatilizing compounds of a liquid composition in a liquid reservoir of the aerosol generating device. The generated aerosol is then guided in a second step 22 to a mouthpiece of the aerosol generating device, the mouthpiece comprising at least two mouthpiece compartments being fluidly separated from each other and each leading from a mouthpiece inlet to a mouthpiece outlet.

In a third step 23 the position of the mouthpiece 10b is aligned with respect to the liquid reservoir, so that in a fourth step 24 the generated aerosol may be guided through at least one of the mouthpiece compartments depending on the aligned position of the mouthpiece with respect to the liquid reservoir. Guiding the aerosol may in each case be effected by an inhaling action of a user of the aerosol generating device through the mouthpiece outlet.

In the foregoing detailed description, various features are grouped together in one or more examples or examples with the purpose of streamlining the disclosure. It is to be understood that the above description is intended to be illustrative, and not restrictive. It is intended to cover all alternatives, modifications and equivalents. Many other examples will be apparent to one skilled in the art upon reviewing the above specification.

The embodiments were chosen and described in order to best explain the principles of the invention and its practical applications, to thereby enable others skilled in the art to best utilize the invention and various embodiments with various modifications as are suited to the particular use contemplated. In the appended claims and throughout the specification, the terms "including" and "in which" are used as the plain-English equivalents of the respective terms "comprising" and "wherein," respectively. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects. Terminology used hereinabove to specify geometric or spatial orientation such as "left", "right", "top", "bottom", "side", "front", "back" and the like is not intended to impose any specific limitation as to the orientation of the objects denoted but rather serve to more easily identify the respective features in the drawings.

### List of Reference Signs

- 1: Liquid interface
- 2: Airflow channel
- 3: Connector fitting
- 4: Sealing ring
- 5: Mouthpiece fitting
- 5a: Mouthpiece inlet
- 6a: Alignment indicator
- 6b: Alignment indicator
- 7a: Alignment mark
- 7b: Alignment mark
- 8: Mouthpiece outlet
- 8a: Partitioning element
- 9a: Mouthpiece compartment
- 9b: Mouthpiece compartment
- 9c: Mouthpiece compartment
- 9d: Mouthpiece compartment
- 10: Aerosol transferring adapter
- 10a: Liquid storage component
- 10b: Mouthpiece
- 11: Liquid reservoir
- 11a: Liquid
- 20: Method
- 21: Method step
- 22: Method step
- 23: Method step
- 24: Method step
- 100: Aerosol generating device
- 101: Power source
- 102: Heating element
- 103: Housing
- 104: Gripping recess
- 105: Air inlet
- F: Airflow

## Claims

1. An aerosol transferring adapter (10) for an aerosol generating device (100), comprising:
a liquid storage component (10a) comprising:
a liquid reservoir (11) for holding a liquid (11a) used to generate an aerosol, an airflow channel (2) leading from the liquid reservoir (11), and a connector fitting (3) arranged around the airflow channel (2); and
a mouthpiece (10b) comprising at least two mouthpiece compartments (9a; 9b; 9c; 9d) being fluidly separated from each other and each leading from at least one mouthpiece inlet (5a) to at least one mouthpiece outlet (8),
wherein at least one of the at least two mouthpiece compartments (9a; 9b; 9c; 9d) is provided with an aerosol-modifying medium,
and a mouthpiece fitting (5) arranged around the mouthpiece inlet (5a), the mouthpiece fitting (5) being configured to be alignably connected to the connector fitting (3) of the liquid storage component (10a), and
the mouthpiece (10b) being further configured to be alignable with respect to the liquid storage compartment (10a) such that an airflow (F) may be selectively directed towards one of the at least two mouthpiece compartments (9a, 9b; 9c; 9d).

2. The aerosol transferring adapter (10) according to claim 1, further comprising:
at least one sealing ring (4) fitted between the connector fitting (3) and the mouthpiece fitting (5) fluidly sealing the interface between the liquid storage component (10a) and the mouthpiece (10b) against the environment.

3. The aerosol transferring adapter (10) according to one of the claims 1 and 2, wherein the connector fitting (3) and the mouthpiece fitting (5) realize complementary snap-fit parts or interlocking clearances.

4. The aerosol transferring adapter (10) according to one of the claims 1 to 3, wherein the mouthpiece (10b) is rotatable against the liquid storage component (10a), the rotation causing the airflow channel (2) to connect to a different compartment of the at least two mouthpiece compartments (9a; 9b; 9c; 9d) depending on the rotation position of the mouthpiece (10b).

5. The aerosol transferring adapter (10) according to claim 4, further comprising:
one or more alignment marks (7a; 7b) in the outer housing of the mouthpiece (10b) and/or the liquid storage component (10a) configured to indicate the rotation position of the mouthpiece (10b) to a user of the aerosol generating device (100).

6. An aerosol generating device (100), comprising:
an aerosol transferring adapter (10) according to one of the claims 1 to 5; and
a heating element (102) coupled to the liquid reservoir (11) at a liquid interface (1) and configured to heat liquid (11a) in the liquid reservoir (11), thereby generating aerosol in the airflow channel (2).

7. The aerosol generating device (100) according to claim 6, further comprising:
an electrical power source (101) coupled to the heating element (102) and configured to provide the heating element (102) with electrical energy.

8. The aerosol generating device (100) according to claim 7, further comprising:
a housing (103) enclosing at least the heating element (102) and the electrical power source (101).

9. A method (20) for transferring aerosol within an aerosol generating device (100), comprising:
- generating (21) aerosol by volatilizing compounds of a liquid composition in a liquid reservoir (11) of the aerosol generating device (100);
- guiding (22) the generated aerosol to a mouthpiece (10b) of the aerosol generating device (100), the mouthpiece (10b) comprising at least two mouthpiece compartments (9a; 9b; 9c; 9d) being fluidly separated from each other and each leading from a mouthpiece inlet (5a) to a mouthpiece outlet;
wherein at least one of the at least two mouthpiece compartments (9a; 9b; 9c; 9d) is provided with an aerosol-modifying medium,
- selectively aligning (23) the position of the mouthpiece (10b) with respect to the liquid reservoir (11); and
- guiding (24) the generated aerosol through at least one of the mouthpiece compartments (9a; 9b; 9c; 9d) depending on the selected aligned position of the mouthpiece (10b) with respect to the liquid reservoir (11).

## Patentansprüche

1. Aerosol-Übertragungsadapter (10) für eine Aerosolerzeugungsvorrichtung (100), umfassend:
eine Flüssigkeitsspeicherkomponente (10a), umfassend:
einen Flüssigkeitstank (11) zum Aufbewahren einer Flüssigkeit (11a), die verwendet wird, um ein Aerosol zu erzeugen, einen Luftstromkanal (2), der von dem Flüssigkeitstank (11) wegführt, und einen Anschlussstutzen (3), der um den Luftstromkanal (2) herum angeordnet ist; und
ein Mundstück (10b), umfassend mindestens zwei Mundstückabteile (9a; 9b; 9c; 9d), die flüssigkeitsbezogen voneinander getrennt sind und wobei jedes davon von mindestens einem Mundstückeinlass (5a) zu mindestens einem Mundstückauslass (8) hinführt,
wobei mindestens eines der mindestens zwei Mundstückabteile (9a; 9b; 9c; 9d) mit einem aerosolmodifizierenden Medium bereitgestellt ist,
und einen Mundstückstutzen (5), der um den Mundstückeinlass (5a) herum angeordnet ist, wobei der Mundstückstutzen (5) konfiguriert ist, bündig mit dem Anschlussstutzen (3) der Flüssigkeitsspeicherkomponente (10a) verbunden zu sein, und
wobei das Mundstück (10b) weiter konfiguriert ist, in Bezug auf das Flüssigkeitsspeicherabteil (10a) ausrichtbar zu sein, sodass ein Luftstrom (F) selektiv in Richtung eines der mindestens zwei Mundstückabteile (9a; 9b; 9c; 9d) gerichtet werden kann.

2. Aerosol-Übertragungsadapter (10) nach Anspruch 1, weiter umfassend:
mindestens einen Dichtungsring (4), der zwischen dem Anschlussstutzen (3) und dem Mundstückstutzen (5) eingepasst ist und die Schnittstelle zwischen der Flüssigkeitsspeicherkomponente (10a) und dem Mundstück (10b) gegen die Umgebung flüssigkeitsbezogen abdichtet.

3. Aerosol-Übertragungsadapter (10) nach einem der Ansprüche 1 und 2, wobei der Anschlussstutzen (3) und der Mundstückstutzen (5) gegenseitig ergänzende Schnappverschlussteile oder Verschlussspielräume realisieren.

4. Aerosol-Übertragungsadapter (10) nach einem der Ansprüche 1 bis 3, wobei das Mundstück (10b) gegen die Flüssigkeitsspeicherkomponente (10a) drehbar ist, wobei die Drehung verursacht, dass sich der Luftstromkanal (2) je nach der Drehposition des Mundstücks (10b) mit einem anderen Abteil der mindestens zwei Mundstückabteile (9a; 9b; 9c; 9d) verbindet.

5. Aerosol-Übertragungsadapter (10) nach Anspruch 4, weiter umfassend:
eine oder mehrere Justierungsmarken (7a; 7b) in dem Außengehäuse des Mundstücks (10b) und/oder wobei die Flüssigkeitsspeicherkomponente (10a) konfiguriert ist, einem Benutzer der Aerosolerzeugungsvorrichtung (100) die Drehposition des Mundstücks (10b) anzuzeigen.

6. Aerosolerzeugungsvorrichtung (100), umfassend:
einen Aerosol-Übertragungsadapter (10) nach einem der Ansprüche 1 bis 5; und
ein Heizelement (102), das an den Flüssigkeitstank (11) an einer Flüssigkeitsschnittstelle (1) gekoppelt und konfiguriert ist, Flüssigkeit (11a) in dem Flüssigkeitstank (11) zu erwärmen, wodurch Aerosol in dem Luftstromkanal (2) erzeugt wird.

7. Aerosolerzeugungsvorrichtung (100) nach Anspruch 6, weiter umfassend:
eine elektrische Stromquelle (101), die an das Heizelement (102) gekoppelt und konfiguriert ist, elektrische Energie an das Heizelement (102) bereitzustellen.

8. Aerosolerzeugungsvorrichtung (100) nach Anspruch 7, weiter umfassend:
ein Gehäuse (103), das mindestens das Heizelement (102) und die elektrische Stromquelle (101) umschließt.

9. Verfahren (20) zum Übertragen von Aerosol innerhalb einer Aerosolerzeugungsvorrichtung (100), umfassend:
- Erzeugen (21) von Aerosol durch verflüchtigende Verbindungen einer Flüssigkeitszusammensetzung in einem Flüssigkeitstank (11) der Aerosolerzeugungsvorrichtung (100);
- Führen (22) des erzeugten Aerosols an ein Mundstück (10b) der Aerosolerzeugungsvorrichtung (100), wobei das Mundstück (10b) mindestens zwei Mundstückabteile (9a; 9b; 9c; 9d) umfasst, die flüssigkeitsbezogen voneinander getrennt sind und wobei jedes davon von einem Mundstückeinlass (5a) zu einem Mundstückauslass hinführt;
wobei mindestens eines der mindestens zwei Mundstückabteile (9a; 9b; 9c; 9d) mit einem aerosolmodifizierenden Medium bereitgestellt ist,
- Selektives Ausrichten (23) der Position des Mundstücks (10b) in Bezug auf den Flüssigkeitstank (11); und
- Führen (24) des erzeugten Aerosols durch mindestens eines der Mundstückabteile (9a; 9b; 9c; 9d), je nach der ausgewählten ausgerichteten Position des Mundstücks (10b) in Bezug auf den Flüssigkeitstank (11).

## Revendications

1. Adaptateur de transfert d'aérosol (10) pour un dispositif de génération d'aérosol (100), comprenant :
un composant de stockage de liquide (10a) comprenant :
un réservoir de liquide (11) pour contenir un liquide (11a) utilisé pour générer un aérosol, un canal d'écoulement d'air (2) s'étendant depuis le réservoir de liquide (11) et un raccord de connexion (3) agencé autour du canal d'écoulement d'air (2) ; et
un embout (10b) comprenant au moins deux compartiments d'embout (9a ; 9b ; 9c ; 9d) qui sont séparés fluidiquement l'un de l'autre et chacun conduisant d'au moins une entrée d'embout (5a) à au moins une sortie d'embout (8),
dans lequel au moins un parmi les au moins deux compartiments d'embout (9a ; 9b ; 9c ; 9d) est pourvu d'un support de modification d'aérosol,
et un raccord d'embout (5) agencé autour de l'entrée d'embout (5a), le raccord d'embout (5) étant configuré pour être connecté, de manière à pouvoir être aligné, au raccord de connexion (3) du composant de stockage de liquide (10a), et
l'embout (10b) étant en outre configuré pour pouvoir être aligné par rapport au composant de stockage de liquide (10a) de sorte qu'un écoulement d'air (F) puisse être dirigé sélectivement vers un parmi les au moins deux compartiments d'embout (9a ; 9b ; 9c; 9d).

2. Adaptateur de transfert d'aérosol (10) selon la revendication 1, comprenant en outre :
au moins une bague d'étanchéité (4) ajustée entre le raccord de connexion (3) et le raccord d'embout (5) scellant fluidiquement l'interface entre le composant de stockage de liquide (10a) et l'embout (10b) contre l'environnement.

3. Adaptateur de transfert d'aérosol (10) selon l'une des revendications 1 et 2, dans lequel le raccord de connexion (3) et le raccord d'embout (5) réalisent des parties à encliqueter complémentaires ou des dégagements d'enclenchement.

4. Adaptateur de transfert d'aérosol (10) selon l'une des revendications 1 à 3, dans lequel l'embout (10b) est rotatif contre le composant de stockage de liquide (10a), la rotation amenant le canal d'écoulement d'air (2) à se connecter à un compartiment différent parmi les au moins deux compartiments d'embout (9a ; 9b ; 9c ; 9d) en fonction de la position de rotation de l'embout (10b).

5. Adaptateur de transfert d'aérosol (10) selon la revendication 4, comprenant en outre :
une ou plusieurs marques d'alignement (7a ; 7b) dans le boîtier extérieur de l'embout (10b) et/ou le composant de stockage de liquide (10a) configurées pour indiquer la position de rotation de l'embout (10b) à un utilisateur du dispositif de génération d'aérosol (100).

6. Dispositif de génération d'aérosol (100), comprenant :
un adaptateur de transfert d'aérosol (10) selon l'une des revendications 1 à 5 ; et
un élément chauffant (102) couplé au réservoir de liquide (11) à une interface liquide (1) et configuré pour chauffer un liquide (11a) dans le réservoir de liquide (11), en générant de ce fait un aérosol dans le canal d'écoulement d'air (2).

7. Dispositif de génération d'aérosol (100) selon la revendication 6, comprenant en outre :
une source d'énergie électrique (101) couplée à l'élément chauffant (102) et configurée pour alimenter l'élément chauffant (102) en énergie électrique.

8. Dispositif de génération d'aérosol (100) selon la revendication 7, comprenant en outre :
un boîtier (103) renfermant au moins l'élément chauffant (102) et la source d'énergie électrique (101).

9. Procédé (20) de transfert d'aérosol à l'intérieur d'un dispositif de génération d'aérosol (100), comprenant :
- la génération (21) d'un aérosol par la volatilisation de composés d'une composition liquide dans un réservoir de liquide (11) du dispositif de génération d'aérosol (100) ;
- le guidage (22) de l'aérosol généré vers un embout (10b) du dispositif de génération d'aérosol (100), l'embout (10b) comprenant au moins deux compartiments d'embout (9a ; 9b ; 9c ; 9d) qui sont séparés fluidiquement l'un de l'autre et chacun conduisant d'une entrée d'embout (5a) à une sortie d'embout ;
dans lequel au moins un parmi les au moins deux compartiments d'embout (9a ; 9b ; 9c ; 9d) est pourvu d'un support de modification d'aérosol,
- l'alignement sélectif (23) de la position de l'embout (10b) par rapport au réservoir de liquide (11) ; et
- le guidage (24) de l'aérosol généré à travers au moins un des compartiments d'embout (9a ; 9b ; 9c ; 9d) en fonction de la position alignée sélectionnée de l'embout (10b) par rapport au réservoir de liquide (11).
